**Europäisches Patentamt**

**European Patent Office**

**Office européen des brevets**

(19)

(11) Veröffentlichungsnummer : **0 327 932 B1**

(12) # EUROPÄISCHE PATENTSCHRIFT

(45) Veröffentlichungstag der Patentschrift :
**15.07.92 Patentblatt 92/29**

(51) Int. Cl.$^5$ : **A61L 9/12**

(21) Anmeldenummer : **89101660.2**

(22) Anmeldetag : **01.02.89**

(54) **Verfahren und Vorrichtung zum kontinuierlichen Verdunsten flüchtiger Wirkstoffe.**

(30) Priorität : **06.02.88 DE 3803665**

(43) Veröffentlichungstag der Anmeldung :
**16.08.89 Patentblatt 89/33**

(45) Bekanntmachung des Hinweises auf die
Patenterteilung :
**15.07.92 Patentblatt 92/29**

(84) Benannte Vertragsstaaten :
**CH DE ES FR GB IT LI NL**

(56) Entgegenhaltungen :
**EP-A- 0 220 352**
**US-A- 1 981 765**

(73) Patentinhaber : **HAARMANN & REIMER GMBH**
**Postfach 138**
**W-3450 Holzminden (DE)**

(72) Erfinder : **Wrede, Wolfgang**
**Danziger Strasse 15**
**W-3450 Holzminden (DE)**
Erfinder : **Rohde, Norbert Dr.**
**Kurze Breite 10**
**W-3457 Deensen (DE)**

(74) Vertreter : **Zobel, Manfred, Dr. et al**
**c/o BAYER AG Konzernverwaltung RP Patente**
**Konzern**
**W-5090 Leverkusen 1 Bayerwerk (DE)**

## Beschreibung

Die Erfindung betrifft ein neues Verfahren zum kontinuierlichen Verdunsten flüchtiger Wirkstoffe, insbesondere von Parfümölen und eine Vorrichtung zur Durchführung des Verfahrens.

Es sind bereits verschiedene Verfahren zum kontinuierlichen Verdunsten flüchtiger Wirkstoffe, z.B. von Parfümölen bekannt. Diese bislang bekannten Verfahren beruhen darauf, daß man entweder durch Vergrößerung der Oberfläche oder aber durch Erhöhung der Temperatur der zu verdunstenden Stoffe eine möglichst gleichmäßige Abgabe der Wirkstoffe an die Umgebungsluft zu erreichen versucht. Zur Vergrößerung der Oberfläche wurden die Wirkstoffe z.B. auf Träger wie Filterpapier, Vliese, geschäumte Kunststoffplatten oder Pulver aus anorganischen Materialien, z.B. Gipspulver, aufgebracht oder in Träger wie Gelatine, vernetzte Cellulosen, Wachse, Polyvinylchlorid oder Ethylen-Vinylacetat-Copolymere eingearbeitet (siehe z.B. US-PS 2 927 055 und 3 945 950; DE-OS 2 454 969, 2 521 265 und 2 606 544). Die Erhöhung der Temperatur wurde durch Zufuhr von Wärme zu den die zu verdampfenden Wirkstoffe enthaltenden Vorratsgefäße bewirkt.

Diese bekannten Verfahren haben jedoch den schwerwiegenden Nachteil, daß die Verdunstung auch bei sorgfältiger Abstimmung der einzelnen Verfahrenskomponenten, z.B. der Träger auf die zu verdunstenden Wirkstoffe, zumindest über längere Zeiträume hinweg nicht linear verläuft, sondern daß sich während des Verdunstungsvorganges die Wirkstoffmenge, die je Zeiteinheit an die Umgebung abgegeben wird und - bei zu verdunstenden Stoffgemischen -auch die Zusammensetzung des gasförmigen Stoffgemisches ändert. Außerdem wird in der Praxis keine quantitative Verdunstung der zu verdunstenden Wirkstoffe erreicht, sondern es entstehen Verdunstungsrückstände, die bis zu 15 Gew.-% der zu verdunstenden Wirkstoffmenge betragen können.

Da in der Praxis der Raumluftverbesserung und der Raumdesinfektion usw. sowohl eine konstante Stoffabgabe als auch eine konstante Stoffzusammensetzung der verdunsteten Stoffgemische verlangt wird, bestand daher die Aufgabe ein Verfahren aufzufinden, mit dessen Hilfe eine lineare Verdunstung der zu verdunstenden Wirkstoffe und Wirkstoffgemische erreicht wird.

Überraschender Weise wurde gefunden, daß man eine solche lineare Verdunstung von flüchtigen Wirkstoffen und Wirkstoffgemischen auf einfache Weise dadurch erreicht, daß man die zu verdunstenden Wirkstoffe bzw. Wirkstoffgemische mittels eines elektrolytisch erzeugten Gases aus ihren Vorratsbehältern fördert.

Es wurde gefunden, daß sich elektrolytisch sowohl kleinste aber auch größere, konstante, exakt reproduzierbare Gasmengen erzeugen lassen und daß sich mit Hilfe dieser exakt reproduzierbaren Gasmengen auch exakt reproduzierbare Wirkstoffmengen aus den Vorratsbehältern fördern und verdunsten lassen. Mit Hilfe des elektrolytisch erzeugten Gasstromes wird erfindungsgemäß ein sowohl zeitlich als auch in seiner Zusammensetzung konstanter Strom gasförmiger Wirkstoffe und damit die gewünschte lineare Verdunstung der flüchtigen Wirkstoffe und Wirkstoffgemische erreicht.

Die Erfindung betrifft daher ein Verfahren zum Verdunsten von flüchtigen Wirkstoffen und Wirkstoffgemischen, das dadurch gekennzeichnet ist, daß man den Wirkstoff oder das Wirkstoffgemisch mit Hilfe eines elektrolytisch erzeugten Gasstromes aus seinem Vorratsgefäß in den mit verdunstendem Wirkstoffgemisch zu versorgenden Raum fördert und dort unmittelbar oder mit Hilfe inerter Verdunster, d.h. Verdunstern, die keine Wechselwirkung mit den Wirkstoffen eingehen, verdunsten läßt.

Die elektrolytische Erzeugung von Gasen, z.B. Wasserstoff, Sauerstoff und Knallgas (2:1-Gemisch von $H_2/O_2$) ist bekannt (siehe Hoffmann'scher Wasserzersetzungsapparat). Bei der Elektrolyse von Wasser scheidet sich an der Kathode Wasserstoff und an der Anode (als Sekundärprodukt) Sauerstoff ab. Sowol der Wasserstoff als auch der Sauerstoff können erfindungsgemäß als Fördergas verwendet werden; vorzugsweise wird jedoch aus Einfachheitsgründen das bei der Elektrolyse entstandene Knallgas unmittelbar verwendet.

Die abgeschiedenen Gasmengen (m) lassen sich unmittelbar nach dem Faraday'schen Gesetz berechnen:

$$m = I \times A \times t.$$

(A = elektrochemisches Äquivalent; I = Stromstärke; t = Zeit).

Die elektrochemischen Äquivalente A betragen für Wasserstoff, Sauerstoff und Knallgas

$O_2$     0,0829 [mg] = 0,0058 [N cm$^3$/As]

$H_2$     0,01044 [mg] = 0,117 [N cm$^3$/As]

Knallgas     0,09334 [mg] = 0,174 [N cm$^3$/As].

Aus den nach dem Faraday'schen Gesetz berechenbaren Mengen an elektrolytisch erzeugtem Gas folgt unmittelbar die Menge an durch diese Gasmenge aus dem Vorratsgefäß geförderter Wirkstoffmenge [ml]. Auf diese Weise läßt sich unmittelbar aus der über die Elektrolyse zugeführten elektrischen Energie (Stromstärke x Zeit) die verdunstende Wirkstoffmenge [ml] bzw. aus der Wirkstoffmenge, die man verdunsten möchte, die erforderliche Gasmenge [ml] und aus dieser die erforderliche elektrische Energie berechnen.

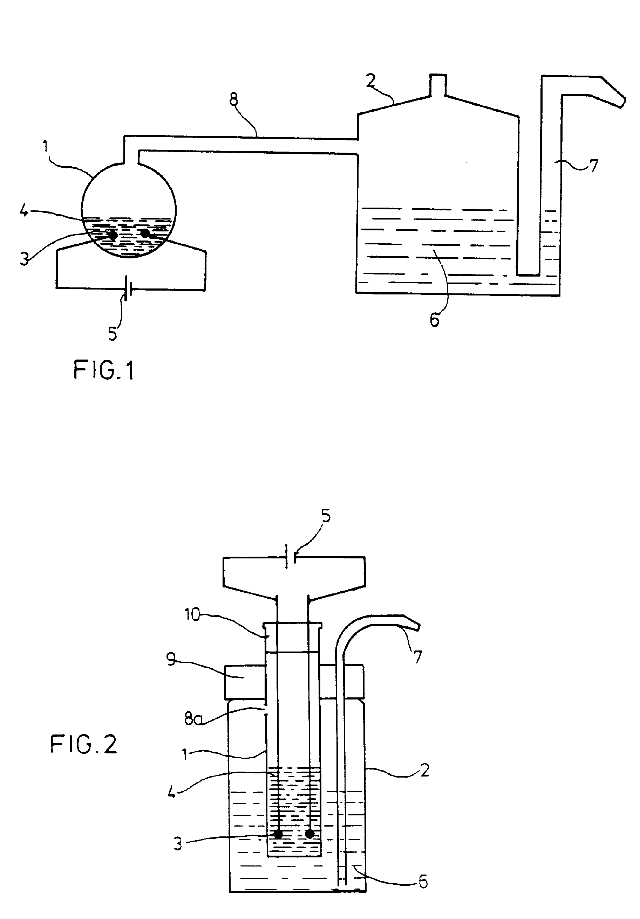

Für die Durchführung des erfindungsgemäßen Verfahrens benötigt man eine Vorrichtung, die im Prinzip nur aus einer Elektrolysezelle zur Erzeugung des Fördergases (Knallgases) und aus einem Vorratsgefäß, besteht, in dem sich der zu verdunstende Wirkstoff oder das zu verdunstende Wirkstoffgemisch befindet. Das Prinzip einer solchen Vorrichtung zur Durchführung des erfindungsgemäßen Verfahrens ist in Fig. 1 dargestellt. In dieser Fig. 1 bedeuten (1) die Elektrolysezelle und (2) das Vorratsgefäß für den zu verdunstenden Wirkstoff. Die Elektrolysezelle enthält 2 Elektroden (3) und z.B. mit $H_2SO_4$ angesäuertes Wasser (4). (5) ist die Stromquelle, mit der die Zelle betrieben wird. Das sich bei der Elektrolyse entwickelnde Knallgas wird über die Leitung (8) in das Vorratsgefäß (2) eingeführt. Dort drückt das Gas auf die Oberfläche des zu verdunstenden flüchtigen Wirkstoffs (6) bzw. Lösungs des Wirkstoffs und fördert ihn (sie) auf diese Weise durch die Austragsleitung (7) in den mit dem verdunstenden Wirkstoffgemisch zu versorgenden Raum.

Die Größen von Elektrolysezelle und Vorratsgefäß ergeben sich aus der Menge an Wirkstoff(gemisch), die in der Zeiteinheit an die Umgebung abgegeben werden soll und den Intervallen, in dem diese Vorrichtung gewartet werden soll (Nachfüllen des zu verdunstenden Wirkstoffs, Nachfüllen des zu elektrolysierenden Wassers). Die berechnung der Größen von Elektrolysezelle und Vorratsgefäß sei an folgendem Beispiel erläutert: Es soll z.B. die Luft in einem Raum, z.B. einem Wartezimmer einer Arztpraxis (Raumgröße: 7 x 4 x 2,30 $m^3$ = 64,5 $m^3$) verbessert werden. Zu diesem Zweck sollen $j_e$ Zeiteinheit (z.B. je Stunde) 0,06 ml Parfümöl im Raum verdunsten. Für die Verdunstung dieser Menge sind 0,06 ml Knallgas und demzufolge (bei einer für die Elektrolyse des Wassers erforderlichen Spannung von ∼ 1,8 bis 3,0 Volt) eine elektrische Energie von (Stromstärke x Zeit) 0,34 As erforderlich.

Als Raumluftverbesserer könnte in diesem Fall, die in Fig. 2 beschriebene, besonders handliche und praktische Vorrichtung verwendet werden. Sie besteht aus einem Vorratsgefäß (2) (Volumen: 30 ml), in das konzentrisch eine kleine Elektrolysezelle (1) eingelassen ist (Volumen der Elektrolysezelle: 3 ml; Größe der einzelnen Elektroden (3): ∼ 5 $mm^2$; Elektrodenmaterial: Pt; Elektroloytmenge (mit $H_2SO_4$ angesäuertes Wasser): 2 ml). Das in der Elektrolysezelle (1) gebildete Gas tritt durch die Gasaustrittsöffnung (8a) in das Vorratsgefäß (2) und drückt aus diesem das Parfümöl (6) durch die Wirstoffaustragskapillare (7) in den Raum.

Die Elektrolysezelle (1) ist durch den Verschluß (10), das Vorratsgefäß (2) durch den Verschluß (9) gasdicht verschlossen. Verschluß (10) dient zugleich als Halterung für die Elektroden (3) und die Stromzuführung.

Die zu verdunstenden Mengen an Wirkstoff hängen von seiner Wirksamkeit (bei Parfümölen: von der Geruchsintensität) ab. Bei Parfümölen betragen die zur Raumluftverbesserung anzuwendenden Mengen für einen Zeitraum von 4 bis 8 Wochen je Raum erfindungsgemäß etwa 50 bis 100 ml Parfümöl, d.h. ∼ 0,06 ml je Raum und Stunde.

Die für die elektrolytische Erzeugung des Förder-Gases erforderliche Energie kann der Elektrolysezelle aus üblichen Stromquellen, z.B. aus Batterien oder aus netzgespeisten Gleichstromquellen, zugeführt werden. Die für die Elektrolyse des Wassers erforderliche Spannung beträgt theoretisch 1,23 Volt, in der Praxis sind jedoch Spannungen von 1,8 bis 2,6 Volt erforderlich. Mit zwei Batterien von je 1,5 Volt und einer Kapazität von 1 Ah lassen sich 627 ml Knallgas erzeugen und damit 627 ml Wirkstoff verdunsten.

Wegen des geringen Energieverbrauchs lassen sich als Stromquellen auch Solarbatterien verwenden. Diese Batterien sind insbesondere dann von Vorteil, wenn eine Verdunstung der Wirkstoffe nur bei Licht, d.h. wenn die Räume bewohnt sind, gewünscht wird.

Den aus dem Vorratsgefäß geförderten Wirkstoff kann man entweder unmittelbar verdunsten lassen oder aber mit Hilfe üblicher inerter, d.h. keine Wechselwirkung mit den zu verdunstenden Wirkstoffen eingehenden Verdunster, z.B. von Ventilatoren angefächelte Platten, im Raum verteilen. Sowohl bei unmittelbarem als auch bei mittelbarem Verdunsten wird eine lineare Verdunstung der Wirkstoffe erreicht.

Unter flüchtigen Wirkstoffen bzw. Wirkstoffgemischen sind im Rahmen des erfindungsgemäßen Verfahrens solche Wirkstoffe und Wirkstoffgemische zu verstehen, die bei Raumtemperatur einen gewissen, für das Verdunsten erforderlichen Dampfdruck aufweisen oder bei denen der erforderliche Dampfdruck durch Erwärmen erzeugt werden kann. Solche flüchtigen Wirkstoff(e) (Gemische) sind z.B. Insektizide wie DDVP, Lindane und Dieldrin; Desinfektionsmittel wie Di- und Triethylenglykol; und insbesondere Parfümöle. Parfümöle sind Stoffgemische, deren Komponenten sich meistens in ihren Dampfdrucken unterscheiden. Mit Hilfe des erfindungsgemäßen Verfahrens wird es erstmals möglich, Parfümöle über einen längeren Zeitraum zu verdunsten, ohne das eine Veränderung in der Geruchsnote der Öle eintritt. Die Geruchsnoten eines mit Hilfe des erfindungsgemäßen Verfahrens verdunsteten Parfümöls ist von der Verdunstung des ersten bis zu Verdunstung des letzten Tropfens konstant. Nach den bislang angewendeten Verdunstungsverfahren bestimmten zu Beginn der Verdunstung die leichter flüchtigen Komponenten die Geruchsnote des verdunsteten Parfümöls, während gegen Ende des Verdunstens die schwerer flüchtigen Bestandteile die Geruchsnote bestimmten.

Beispiel

Zum Versprühen von 6,2 ml Parfümöl je Stunde, d.h. einer Parfümöl-Menge, die zur Luftverbesserung von Großräumen (Räume mit Volumina von 5000 bis 10 000 m³), z.B. Veranstaltungsräumen, wie Theater- und Konzertsäle oder Großraumbüros ausreicht wird die nachfolgend beschriebene Vorrichtung verwendet; sie ist im Prinzip wie die in Fig. 2 beschriebene Vorrichtung aufgebaut.

Beschreibung der konzentrisch in das Vorratsgefäß (2) eingelassenen Elektrolysezelle (1):
(Glasflasche, Durchmesser: 15 mm; Höhe: 60 mm; Elektrolyt: 3 ml mit einigen Tropfen Schwefelsäure angesäuertes Wasser; Größe der einzelnen Platinelektroden: 5 mm²).

Die Elektrodenanschlüsse werden durch einen Eindrückdeckel (10), der die Glasflasche gasdicht abschließt, geführt und mit einer Solarzelle (Maße: 90 x 90 mm; maximale Spannung: 3 V; maximale Stromstärke: 0,01 A) verbunden.

Bei der Elektrolyse werden in dieser Zelle je Stunde 6,2 ml Knallgas, gebildet. Diese treten durch die Austrittsöffnung (8a) in den Gasraum des Vorratsgefäßes (2) ein.

Beschreibung des Vorratsgefäßes (2): mit Schraubdeckel (9) versehene und mit 75 ml Parfümöl (6) gefüllte 100 ml-Weithals-Glasflasche. Diese Glasflasche ist ferner mit einer als Austrittsleitung dienenden Stahlkapillare (7) (Durchmesser: 0,25 mm) ausgerüstet; diese Kapillare reicht bis kurz über den Boden der Weithals-Glasflasche und wird durch den Schraubdeckel herausführt.

Mit Hilfe der je Stunde erzeugten 6,2 ml Knallgas werden je Stunde 6,2 ml Parfümöl aus dem Vorratsgefäß durch die Kapillare auf die inerte Fläche eines Verdampfers gefördert und von dort verdampft.

**Patentansprüche**

1. Verfahren zum Verdunsten von flüchtigen Wirkstoffen und Wirkstoffgemischen, dadurch gekennzeichnet, daß man den Wirkstoff oder das Wirkstoffgemisch mit Hilfe eines elektrolytisch erzeugten Gases aus seinem Vorratsgefäß in den mit verdunstendem Wirkstoff zu versorgenden Raum fördert und in diesem unmittelbar oder mit Hilfe bekannter inerter Verdunster verdunsten läßt.

2. Verfahren nach Anspruch 1, dadurch gekennzeichnet, daß das elektrolytisch erzeugte Gas Knallgas ist.

3. Verfahren nach Anspruch 1, dadurch gekennzeichnet, daß es zum Verdunsten von Parfümölen angewendet wird.

4. Verfahren gemäß Ansprüchen 1 bis 3, dadurch gekennzeichnet, daß der (das) aus dem Vorratsgefäß geförderte Wirkstoff(gemisch) mittels eines üblichen inerten Verdunsters im Raum verteilt wird.

5. Vorrichtung zur Durchführung des Verfahrens gemäß Ansprüchen 1 bis 4 bestehend aus einer Elektrolysezelle und einem Vorratsgefäß für den (das) zu verdunstende(n) Wirkstoff(gemisch).

6. Vorrichtung gemäß Anspruch 4, dadurch gekennzeichnet, daß die Elektrolysezelle 2 Elektroden und mit Schwefelsäure angesäuertes Wasser enthält und das bei der Elektrolyse entwickelte Knallgas über eine Leitung in das Vorratsgefäß eingeführt wird und dort auf die Oberfläche des zu verdunstenden Wirkstoffes bzw. Wirkstoffgemisches oder der Wirkstofflösung drückt und ihn (es, sie) auf diese Weise durch eine Austragsleitung in den mit dem verdunstenden (Wirkstoff(gemisch) zu versorgenden Raum fördert.

7. Vorrichtung gemäß Ansprüche 5 und 6, dadurch gekennzeichnet, daß sie aus einer Elektrolysezelle, einem Wirkstoffvorratsgefäß und einem üblichen inerten Verdunster besteht.

**Claims**

1. Process for the vaporization of volatile active compounds and mixtures of active compounds, characterised in that the active compound or the mixture of active compounds is conveyed from its storage vessel by means of an electrolytically produced gas into the room which is to be supplied with a vaporizing active compound, and is caused to vaporize in this room directly or by means of known inert vaporizers.

2. Process according to Claim 1, characterised in that the gas produced electrolytically is oxyhydrogen gas.

3. Process according to Claim 1, characterised in that it is used for the vaporization of perfume oils.

4. Process according to Claims 1 to 3, characterised in that the active compound (mixture) conveyed from the storage vessel is distributed within the room by means of a customary inert vaporizer.

5. Device for carrying out the process according to Claims 1 to 4, consisting of an electrolytic cell and a storage vessel for the active compound (mixture) to be vaporized.

6. Device according to Claim 4, characterised in that the electrolytic cell contains 2 electrodes and water which has been acidified with sulphuric acid, and the oxyhydrogen gas evolved in the electrolysis is introduced via a line into the storage vessel and there presses on the surface of the active compound or mixture of active compounds or the solution of active compound to be vaporized and conveys it in this manner through an exit line into the room to be supplied with the vaporizing active compound (mixture).

7. Device according to Claims 5 and 6, characterised in that it consists of an electrolytic cell, a storage vessel for the active compound and a customary inert vaporizer.

## Revendications

1. Procédé pour vaporiser des substances actives et mélanges de substances actives volatils, caractérisé en ce que l'on transporte la substance active ou le mélange de substances actives à l'aide d'un gaz produit par électrolyse hors de son réservoir dans le local à alimenter en la substance active vaporisée dans lequel elle vaporise spontanément ou est vaporisée au moyen d'évaporateurs inertes connus.

2. Procédé selon la revendication 1, caractérisé en ce que le gaz produit par électrolyse est le gaz détonant.

3. Procédé selon la revendication 1, caractérisé en ce qu'on l'exploite pour la vaporisation de parfums.

4. Procédé selon les revendications 1 à 3, caractérisé en ce que la substance active ou le mélange de substances actives transporté hors du réservoir est réparti dans le local à l'aide d'un évaporateur inerte usuel.

5. Appareillage pour la mise en oeuvre du procédé selon les revendications 1 à 4, consistant en une cellule d'électrolyse et un réservoir pour la substance active ou le mélange de substances actives à vaporiser.

6. Appareillage selon la revendication 4, caractérisé en ce que la cellule d'électrolyse (2) contient des électrodes et de l'eau acidifiée par de l'acide sulfurique, le gaz détonant produit par l'électrolyse est envoyé par un conduit dans le réservoir où il presse à la surface de la substance active ou du mélange de substances actives ou de la solution de substance active à vaporiser et les refoule, au travers d'un conduit de sortie, dans le local à approvisionner en la substance active ou en le mélange de substances actives à vaporiser.

7. Appareillage selon les revendications 5 et 6, caractérisé en ce qu'il consiste en une cellule d'électrolyse, un réservoir de substance active et un évaporateur inerte usuel.

FIG.1

FIG.2